# EUROPEAN PATENT APPLICATION

(11) **EP 3 266 480 A1**
(43) Date of publication of application: **10.01.2018**
(21) Application number: 15884682.4
(22) Date of filing: 26.11.2015
(51) Int. Cl.: A61M 5/31

(54) **BARREL WITH NEEDLE AND SYRINGE PROVIDED WITH SAME**

(30) Priority: 06.03.2015 JP 2015044877
(71) Applicant: Taisei Kako Co., Ltd., Kita-ku Osaka-shi Osaka 531-0072 (JP)
(72) Inventor: HORITA, Taiji, Ibaraki-shi Osaka 567-0054 (JP); TAKAMI, Kouji, Ibaraki-shi Osaka 567-0054 (JP); MATSUMOTO, Ippei, Ibaraki-shi Osaka 567-0054 (JP); TANIGUCHI, Kensuke, Ibaraki-shi Osaka 567-0054 (JP)
(74) Representative: Stolmár & Partner Patentanwälte PartG mbB
(86) International application number: PCT/JP2015/083168
(87) International publication number: WO 2016/143198

(57) **Abstract**

Provided is a barrel with needle including: a barrel body; and an injection needle, wherein the barrel body has a cylindrical body wall, and an end wall through which the injection needle is inserted, and the inner wall surface of the end wall expands in a direction intersecting the axial direction of the body wall and inclined toward the other opening side of the body wall, with a specific position between an outer circumferential edge and an inner circumferential edge on a proximal end surface of the injection needle serving as a starting point.

## Description

### CROSS-REFERENCE TO RELATED APPLICATION

This application claims priority to Japanese Patent Application No. 2015-044877, filed on March 6, 2015, the disclosure of which is incorporated herein by reference in its entirety.

### FIELD

The present invention relates to a barrel with needle including an injection needle, and a syringe including the barrel with needle.

### BACKGROUND

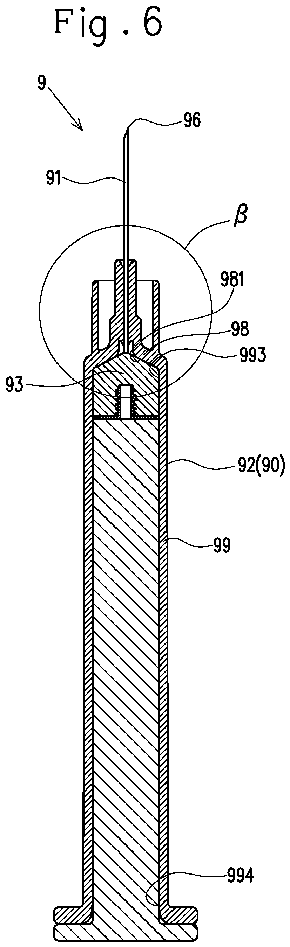

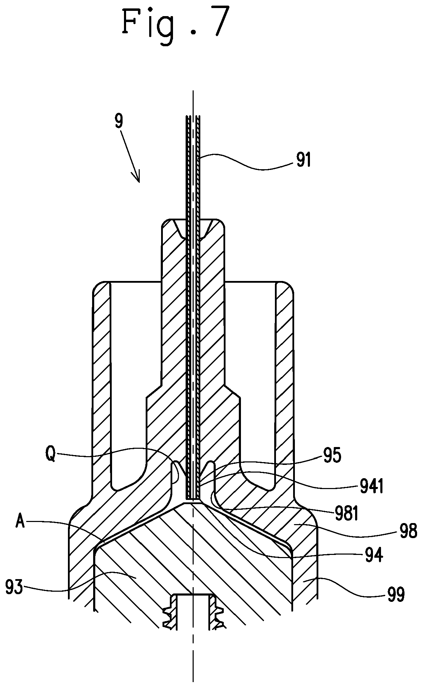

Conventionally, a syringe including a barrel with needle including an injection needle (hereinafter, referred to as syringe) has been provided as one of medical devices used for injection. As shown in Fig. 6 and Fig. 7, a syringe 9 generally includes a barrel with needle 90 including a barrel body 92 configured to contain a formulation and an injection needle 91 with a proximal end 94 opening into the barrel body 92, and a plunger 93 configured to expel the formulation contained in the barrel body 92 to the outside of the barrel with needle 90 through a distal end 96 of the injection needle 91 (Patent Literature 1).

In the barrel with needle 90, the barrel body 92 has a cylindrical body wall 99, and an end wall 98 closing one opening 993 of the body wall 99. The end wall 98 includes an inner wall surface 981 located on the body wall 99 side, and the injection needle 91 is inserted and attached thereto. Thus, the barrel body 92 can contain the formulation.

The plunger 93 is formed into a rod shape with substantially the same outer diameter as the inner diameter of the body wall 99 so as to be movable in the axial direction of the body wall 99. The plunger 93 is inserted into the barrel with needle 90 through the other opening 994 of the body wall 99.

Thus, in the syringe 9 of this type, when the plunger 93 is pushed toward the injection needle 91 side, the formulation in an internal space A defined by the barrel body 92 and the plunger 93 is expelled to the outside of the barrel with needle 90 through the distal end 96 of the injection needle 91, following an increase in pressure in the internal space A. Thus, the formulation is injected to an injection target.

### CITATION LIST

### Patent Literature

Patent Literature 1: WO2008/139982

Meanwhile, in the syringe 9 of this type, the inner wall surface 981 of the end wall 98 expands in the radial direction of the body wall 99 from an outer circumferential surface 95 of a proximal end part 941 of the injection needle 91 while curving toward the distal end 96 side of the injection needle 91 to form a recess. Therefore, the outer circumferential surface 95 of the injection needle 91 and the inner wall surface 981 of the end wall 98 form a recess Q recessed toward the distal end 96 side of the injection needle 91 along the outer circumferential surface 95 of the proximal end part 941 of the injection needle 91.

Accordingly, in the syringe 9 of this type, even if the plunger 93 is pushed into the barrel body 92 to the limit position of pushing, and the formulation contained in the barrel body 92 is expelled to the outside of the barrel with needle 90, the formulation remains in the recess Q.

The syringe 9 used as a medical device is mostly discarded after the use as it is, and therefore in the syringe 9 of this type, the useful formulation is discarded without being injected.

### SUMMARY

### Technical Problem

In view of such an actual situation, it is therefore an object of the present invention to provide a barrel with needle that allows the formulation contained therein to be used without waste, and a syringe including the barrel with needle.

### Solution to Problem

A barrel with needle according to the present invention includes: a barrel body defining an internal space configured to contain a formulation; and an injection needle with its proximal end opening into the internal space of the barrel body, wherein the barrel body includes: a cylindrical body wall; and an end wall closing one opening of the body wall and having an inner wall surface located on the body wall side, the injection needle is inserted and attached to the end wall, and the inner wall surface of the end wall expands in a radial direction of the body wall or in a direction intersecting an axial direction of the body wall and inclined toward the other opening side of the body wall, with any one of an outer circumferential edge, an inner circumferential edge, and a specific position between the outer circumferential edge and the inner circumferential edge on a proximal end surface of the injection needle serving as a starting point.

According to one embodiment of the present invention, the inner wall surface of the end wall may expand in a direction intersecting the axial direction of the body wall and inclined toward the other opening side of the body wall, from the inner circumferential edge or a specific position between the outer circumferential edge and the inner circumferential edge on the proximal end surface of the injection needle.

A syringe according to the present invention includes: the aforementioned barrel with needle; and a plunger configured to be inserted into the barrel with needle and to expel the formulation contained in the barrel body to the outside of the barrel with needle through a distal end of the injection needle.

### BRIEF DESCRIPTION OF DRAWINGS

Fig. 1 is a front view of a syringe according to an embodiment of the present invention.
Fig. 2A is an enlarged partial view of part α of the syringe according to the aforementioned embodiment in Fig. 1.
Fig. 2B is an enlarged partial view of the syringe according to the aforementioned embodiment in Fig. 2A.
Fig. 3A is an enlarged partial view of part α of a syringe according to another embodiment in Fig. 1.
Fig. 3B is an enlarged partial view of the syringe according to the aforementioned embodiment in Fig. 3A.
Fig. 4A is an enlarged partial view of part α of a syringe according to still another embodiment in Fig. 1.
Fig. 4B is an enlarged partial view of the syringe according to the aforementioned embodiment in Fig. 4A.
Fig. 5A is a modification of the barrel with needle according to the present invention.
Fig. 5B is another modification of the barrel with needle according to the present invention.
Fig. 5C is a still another modification of the barrel with needle according to the present invention.
Fig. 6 is a front view of a conventional syringe.
Fig. 7 is an enlarged partial view of part β in Fig. 6.

### DESCRIPTION OF EMBODIMENT

A barrel with needle according to this embodiment includes: a barrel body defining an internal space configured to contain a formulation; and an injection needle with its proximal end opening into the internal space of the barrel body, wherein the barrel body includes: a cylindrical body wall; and an end wall closing one opening of the body wall and having an inner wall surface located on the body wall side, the injection needle is inserted and attached to the end wall, and the inner wall surface of the end wall expands in a radial direction of the body wall or in a direction intersecting an axial direction of the body wall and inclined toward the other opening side of the body wall, with any one of an outer circumferential edge, an inner circumferential edge, and a specific position between the outer circumferential edge and the inner circumferential edge on a proximal end surface of the injection needle serving as a starting point.

In the barrel with needle with such a configuration, the inner wall surface of the end wall is not located more on the distal end side of the injection needle than the proximal end surface of the injection needle. Therefore, a recess recessed toward the distal end side of the injection needle is not formed along the outer circumferential surface of the proximal end part of the injection needle by the outer circumferential surface of the injection needle and the inner wall surface of the end wall. As a result, the formulation does not accumulate in the recess, and the formulation contained in the barrel body can be used without waste.

According to one embodiment of the present invention, the inner wall surface of the end wall may expand in a direction intersecting the axial direction of the body wall and inclined toward the other opening side of the body wall, from the inner circumferential edge or a specific position between the outer circumferential edge and the inner circumferential edge on the proximal end surface of the injection needle.

In the barrel with needle with such a configuration, the inner wall surface of the end wall can partially or entirely cover the proximal end surface of the injection needle. Therefore, even if the distal end of the injection needle is pierced into the injection target, and the injection needle is pressed toward the barrel body side, the inner wall surface of the end wall can receive the proximal end surface of the injection needle. Accordingly, the barrel with needle with such a configuration can allow the injection needle to be firmly inserted and attached.

Further, since the inner wall surface of the end wall partially or entirely covers the proximal end surface of the injection needle, even if the formulation is pushed toward the injection needle side, the total pressure of the formulation is not applied to the proximal end surface of the injection needle. Accordingly, the barrel with needle with such a configuration has a strength that can sufficiently withstand the hydraulic pressure of the formulation since the proximal end surface of the injection needle is less likely to be pressed toward the distal end side of the injection needle.

A syringe according to the present invention includes: the aforementioned barrel with needle; and a plunger configured to be inserted into the barrel with needle and to expel the formulation contained in the barrel body to the outside of the barrel with needle through a distal end of the injection needle.

In the syringe with such a configuration, the inner wall surface of the end wall is not located more on the distal end side of the injection needle than the proximal end surface of the injection needle. Therefore, a recess recessed toward the distal end side of the injection needle is not formed along the outer circumferential surface of the proximal end part of the injection needle by the outer circumferential surface of the injection needle and the inner wall surface of the end wall. Accordingly, the formulation does not accumulate in the recess, and the formulation contained in the barrel body can be used without waste.

Further, in the syringe with such a configuration, the inner wall surface of the end wall can partially or entirely cover the proximal end surface of the injection needle. Therefore, even if the distal end of the injection needle is pierced into the injection target, and the injection needle is pressed toward the barrel body side, the inner wall surface of the end wall can receive the proximal end surface of the injection needle. Accordingly, the syringe with such a configuration can allow the injection needle to be firmly inserted and attached.

Further, since the inner wall surface of the end wall partially or entirely covers the proximal end surface of the injection needle, the total pressure of the formulation is not applied to the proximal end surface of the injection needle when the plunger expels the formulation from the barrel with needle. Accordingly, the syringe with such a configuration has a strength that can sufficiently withstand the hydraulic pressure of the formulation since the proximal end surface of the injection needle is less likely to be pressed toward the distal end side of the injection needle.

As can be seen from the above, this embodiment can provide a barrel with needle and a syringe including the barrel with needle, in which the formulation contained therein can be used without waste.

Hereinafter, a syringe including a barrel with needle according to an embodiment of the present invention will be described with reference to the drawings.

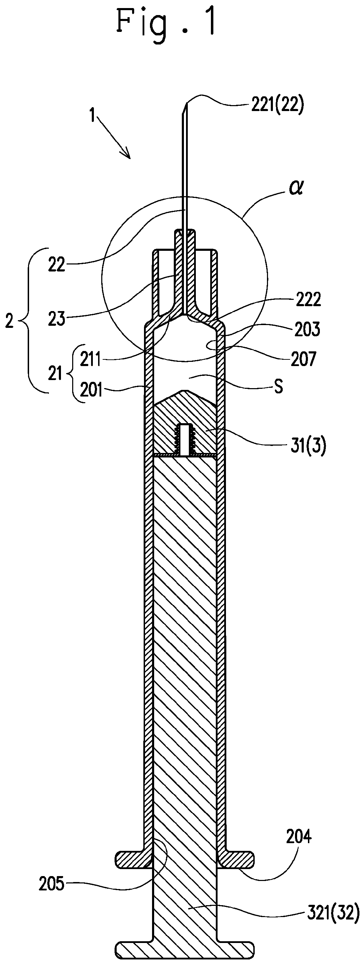

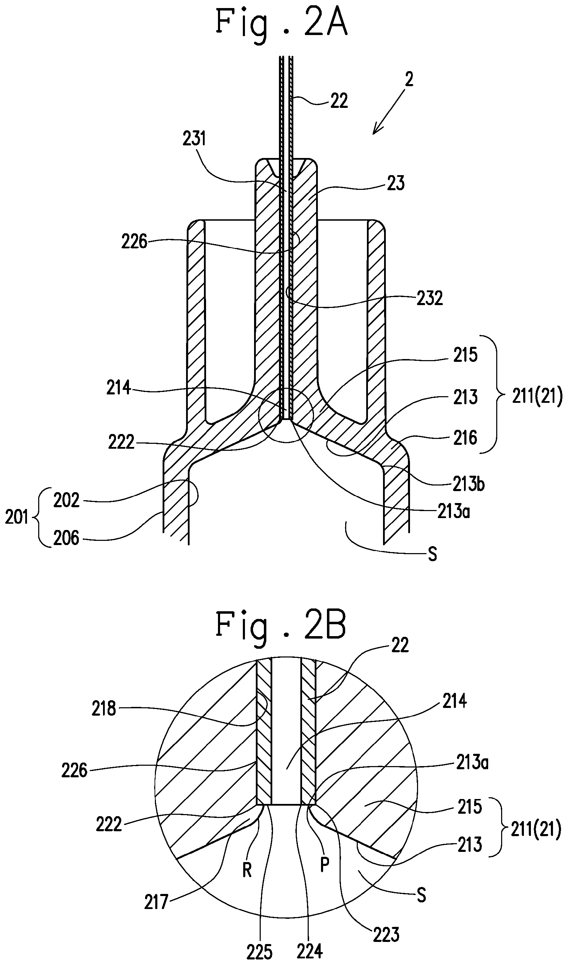

As shown in Figs. 1 and 2, the syringe including the barrel with needle according to this embodiment (hereinafter, referred to as syringe) includes a barrel with needle 2 including a barrel body 21 defining an internal space S configured to contain a formulation and an injection needle 22 with its proximal end 222 opening into the internal space S of the barrel body 21, and a plunger 3 configured to be inserted into the barrel with needle 2 and to expel the formulation contained in the barrel body 21 to the outside of the barrel with needle 2 through a distal end 221 of the injection needle 22. The syringe 1 further includes a cap (not shown) configured to cover the distal end 221 of the injection needle 22.

The barrel with needle 2 further includes a needle fixing part 23 configured to fix the injection needle 22 at the distal end.

The barrel body 21 has a cylindrical body wall 201, and an end wall 211 closing one opening 207 of the body wall 201. The end wall 211 has an inner wall surface 213 located on the body wall 201 side, and the injection needle 22 is inserted and attached thereto.

The barrel body 21 is formed using a rigid material. The barrel body 21 is formed, for example, using a resin such as COP (cycloolefin polymer), COC (cycloolefin copolymer), PP (polypropylene), or PE (polyethylene), or a glass.

The body wall 201 is formed into a cylindrical shape. The body wall 201 is formed with a constant diameter from one end 203 to the other end 204. The one opening 207 of the body wall 201 is closed by the end wall 211, and the other opening 205 is open. Accordingly, the barrel body 21 has one end closed and the other end open, so that the plunger 3 can be inserted thereinto.

The end wall 211 is formed with a specific thickness so as to withstand the hydraulic pressure during injection. In the end wall 211, an insertion hole 214 through which the injection needle 22 is inserted is formed. Accordingly, the end wall 211 is formed into an annular shape. The end wall 211 has a body 215 expanding from the insertion hole 214 toward the end edge of the end wall 211, and an end edge part 216 forming the end edge.

The end wall 211 is formed into a tapered shape with the center of the insertion hole 214 serving as the starting point. Accordingly, the body 215 projects from the end edge part 216 in a direction in which the center line of the insertion hole 214 extends. Further, the end edge part 216 is formed into a substantially circular shape. Accordingly, the inner wall surface 213 of the end wall 211 is an annular surface having an inner circumferential edge 213a and an outer circumferential edge 213b.

The inner wall surface 213 of the end wall 211 expands in the radial direction of the body wall 201 or a direction intersecting the axial direction of the body wall 201 and inclined toward the other opening 205 side of the body wall 201, with any one of an outer circumferential edge 223, an inner circumferential edge 224, and a specific position on a proximal end surface 225 of the injection needle 22 between the outer circumferential edge 223 and the inner circumferential edge 224 serving as a starting point.

The inner circumferential edge 213a of the inner wall surface 213 is located on the proximal end surface 225 of the injection needle 22. Specifically, the inner circumferential edge 213a is located within a region including the outer circumferential edge 223 and the inner circumferential edge 224 on the proximal end surface 225 of the injection needle 22. The outer circumferential edge 213b of the inner wall surface 213 is located at the same position as the inner circumferential edge 213a or is located more on the other opening 205 side of the body wall 201 than the inner circumferential edge 213a, in the axial direction of the body wall 201. Further, the inner wall surface 213 is located at the same position as the outer circumferential edge 223 of the injection needle 22 or is located more on the other opening 205 side of the body wall 201 than the outer circumferential edge 223, in the axial direction of the body wall 201. Thus, the inner wall surface 213 is not located more on the distal end 221 side of the injection needle 22 than the proximal end surface 225 of the injection needle 22.

In this embodiment, the inner wall surface 213 of the end wall 211 expands in a direction intersecting the axial direction of the body wall 201 and inclined toward the other opening 205 side of the body wall 201, from a specific position between the outer circumferential edge 223 and the inner circumferential edge 224 on the proximal end surface 225 of the injection needle 22.

Specifically, as shown in Fig. 2A and Fig. 2B, the inner circumferential edge 213a of the inner wall surface 213 is located between the outer circumferential edge 223 and the inner circumferential edge 224 on the proximal end surface 225 of the injection needle 22. Accordingly, the inner wall surface 213 of the end wall 211 expands from the center of the proximal end surface 225 of the injection needle 22. Thus, the inner wall surface 213 of the end wall 211 constitutes a supporting part 217 configured to support (receive) the proximal end surface 225 of the injection needle 22 in the body 215.

The inner wall surface 213 of the end wall 211 constitutes a curved surface R extending from a connection point P with the injection needle 22 to a specific position. The curved surface R is curved radially inwardly of the insertion hole 214 of the end wall 211 to form a projection. Thereby, the supporting part 217 is formed to be thick. The curved surface R has a specific radius of curvature.

The inner wall surface 213 of the end wall 211 constitutes a smooth surface from the inner circumferential edge 213a to the outer circumferential edge 213b. That is, corners, projections, and the like are not formed on the inner wall surface 213 of the end wall 211.

The injection needle 22 is formed into a cylindrical shape so as to allow the formulation to circulate therethrough. The injection needle 22 is formed with a constant inner diameter and a constant outer diameter from the distal end 221 to the proximal end 222. The proximal end surface 225 of the injection needle 22 is constituted by a flat surface without unevenness from the outer circumferential edge 223 to the inner circumferential edge 224. The proximal end surface 225 of the injection needle 22 is orthogonal to the axial direction of the injection needle 22.

The injection needle 22 has the distal end 221 that is sharpened so as to be capable of piercing an injection target. The injection needle 22 is rigid. The injection needle 22 is formed, for example, by wire drawing of a steel tube made of stainless steel. Further, the injection needle 22 is formed, for example, using aluminum, aluminum alloy, titanium, titanium alloy, and other metals.

The injection needle 22 has a proximal end part inserted and attached into the insertion hole 214 of the end wall 211. The injection needle 22 has an outer circumferential surface 226 in close contact with an inner circumferential surface 218 of the insertion hole 214. The injection needle 22 has the distal end 221 projecting from the needle fixing part 23.

The needle fixing part 23 is provided continuously with the end wall 211. In the needle fixing part 23, an insertion hole 231 into which the injection needle 22 is inserted and attached is formed. The needle fixing part 23 is provided continuously with the end wall 211 on the side on which the body 215 projects.

The needle fixing part 23 is arranged so that the center line of the insertion hole 231, the center line of the insertion hole 214 of the end wall 211, and the axis of the body wall 201 coincide with one another. The insertion hole 231 of the needle fixing part 23 has substantially the same diameter as the insertion hole 214 of the end wall 211. The needle fixing part 23 of this embodiment is formed into a cylindrical shape. The needle fixing part 23 is formed with an outer diameter smaller than the outer diameter of the barrel body 21.

The needle fixing part 23 fixes the injection needle 22 by bringing an inner circumferential surface 232 of the insertion hole 231 into contact with the outer circumferential surface 226 of the injection needle 22.

The needle fixing part 23 is formed using a rigid material. The needle fixing part 23 is formed using the same material as the barrel body 21.

The plunger 3 includes an expelling part 31 configured to be inserted into the barrel with needle 2 and to be slidable within the barrel body 21, and a plunger body 32 having the expelling part 31 at the distal end. The plunger body 32 is formed into a rod shape. The plunger body 32 is formed to be longer than the barrel body 21. That is, when the expelling part 31 is inserted into the barrel body 21 to the limit position of pushing, a proximal end part 321 of the plunger body 32 projects from the opening of the barrel body 21.

The outer diameter of the expelling part 31 is formed to be substantially the same as the inner diameter of the body wall 201 of the barrel body 21. The distal end of the expelling part 31 is formed in conformity with the shape of the inner wall surface 213 of the end wall 211. That is, when the distal end of the plunger 3 is pushed into the barrel body 21 to the limit position of pushing, the distal end of the plunger 3 is fitted to the inner wall surface 213 of the end wall 211.

Since the end wall 211 is formed into a tapered shape, the distal end of the plunger 3 of this embodiment is formed into the tapered shape.

As described above, the barrel with needle 2 according to this embodiment includes: a barrel body 21 defining an internal space S configured to contain a formulation; and an injection needle 22 with its proximal end 222 opening into the internal space S of the barrel body 21, wherein the barrel body 21 includes: a cylindrical body wall 201; and an end wall 211 closing one opening 207 of the body wall 201 and having an inner wall surface 213 located on the body wall 201 side, the injection needle 22 is inserted and attached to the end wall 211, and the inner wall surface 213 of the end wall 211 expands in a direction intersecting the axial direction of the body wall 201 and inclined toward the other opening 205 side of the body wall 201, from an inner circumferential edge 224 or a specific position between an outer circumferential edge 223 and the inner circumferential edge 224 on a proximal end surface 225 of the injection needle 22.

The syringe 1 according to this embodiment includes: the barrel with needle 2; and a plunger 3 configured to be inserted into the barrel with needle 2 and to expel the formulation contained in the barrel body 21 to the outside of the barrel with needle 2 through a distal end 221 of the injection needle 22.

According to the aforementioned configuration, the inner wall surface 213 of the end wall 211 is not located more on the distal end 221 side of the injection needle 22 than the proximal end surface 225 of the injection needle 22. Therefore, a recess recessed toward the distal end 221 side of the injection needle 22 is not formed along the outer circumferential surface 226 of the proximal end part of the injection needle 22 by the outer circumferential surface 226 of the injection needle 22 and the inner wall surface 213 of the end wall 211. As a result, the formulation does not accumulate in the recess, and the formulation contained in the barrel body 21 can be used without waste.

Further, the inner wall surface 213 of the end wall 211 can partially or entirely cover the proximal end surface 225 of the injection needle 22. Therefore, even if the distal end 221 of the injection needle 22 is pierced into the injection target, and the injection needle 22 is pressed toward the barrel body 21 side, the inner wall surface 213 of the end wall 211 can receive the proximal end surface 225 of the injection needle 22. Accordingly, the aforementioned configuration can allow the injection needle 22 to be firmly inserted and attached.

Further, since the inner wall surface 213 of the end wall 211 partially or entirely covers the proximal end surface 225 of the injection needle 22, even if the formulation is pushed toward the injection needle 22 side, the total pressure of the formulation is not applied to the proximal end surface 225 of the injection needle 22 (when the plunger 3 expels the formulation from the barrel with needle 2). Accordingly, the aforementioned configuration gives a strength that can sufficiently withstand the hydraulic pressure of the formulation since the proximal end surface 225 of the injection needle 22 is less likely to be pressed toward the distal end 221 side of the injection needle 22.

According to the aforementioned configuration, the inner wall surface 213 of the end wall 211 expands with any one of the outer circumferential edge 223, the inner circumferential edge 224, and a specific position between the outer circumferential edge 223 and the inner circumferential edge 224 on the proximal end surface 225 of the injection needle 22 serving as a starting point, and therefore the proximal end part of the injection needle 22 is not exposed to the internal space S. Therefore, even if impurities such as oils adhere onto the outer circumferential surface 226 of the injection needle 22 when forming the injection needle 22, such impurities are not entrained into the formulation. Accordingly, injection of such impurities to the injection target together with the formulation can be prevented.

Further, since the proximal end part of the injection needle 22 is not exposed to the internal space S, the hydraulic pressure of the formulation is not applied to the outer circumferential surface 226 of the injection needle 22 when the plunger 3 expels the formulation to the outside of the barrel with needle 2. Accordingly, breaking and cracking are less likely to occur in the injection needle 22.

According to the aforementioned configuration, the inner wall surface 213 of the end wall 211 is not located more on the distal end 221 side of the injection needle 22 than the proximal end surface 225 of the injection needle 22. Therefore, the flow of the formulation is less likely to be disturbed on the proximal end surface 225 of the injection needle 22 when the formulation is expelled from the barrel body 21. Specifically, a recess recessed toward the distal end 221 side of the injection needle 22 is not formed along the outer circumferential surface 226 of the proximal end part of the injection needle 22, and therefore the formulation does not enter the recess. Accordingly, when the formulation is expelled from the barrel body 21, there is no formulation flowing out around the recess, and therefore the formulation can be rectified. Following this, during the expelling from the barrel with needle 2, the resistance in the plunger 3 can be reduced.

According to the aforementioned configuration, the supporting part 217 is formed to be thick, and therefore the strength of the supporting part 217 is increased. Accordingly, the syringe 1 with the aforementioned configuration allows the injection needle 22 to be firmly inserted and attached.

According to the aforementioned configuration, the distal end of the expelling part 31 of the plunger 3 is formed in conformity with the shape of the inner wall surface 213 of the end wall 211. Therefore, when the plunger 3 is inserted into the barrel body 21 to be pushed into the barrel body 21 to the limit position of pushing, most of the formulation contained in the barrel body 21 can be expelled to the outside of the barrel with needle 2.

The barrel with needle 2 and the syringe 1 of the present invention are not limited to the aforementioned embodiment. Further, the barrel with needle 2 and the syringe 1 according to the present invention are not limited to the aforementioned operational effects. In the barrel with needle 2 and the syringe 1 according to the present invention, various modifications can of course be made without departing from the gist of the present invention.

In the aforementioned embodiment, there is no particular mention of the methods for forming the barrel with needle 2 and the syringe 1, the barrel with needle 2 and the syringe 1 according to the present invention can be formed by insert molding by integrally injection-molding the injection needle 22, the needle fixing part 23, and the barrel body 21. Further, the barrel with needle 2 and the syringe 1 according to the present invention can be formed also by a method of integrally injection-molding the needle fixing part 23 and the barrel body 21 in advance, thereafter inserting the injection needle 22 into the needle fixing part 23, and fixing the injection needle 22 and the needle fixing part 23 together with an adhesive or by heat welding.

In the case of integrally forming the injection needle 22, the needle fixing part 23, and the barrel body 21 together by insert molding, a gap is less likely to be formed between the injection needle 22 and the needle fixing part 23. That is, in insert molding, the barrel with needle 2 is formed by allowing a resin or the like to flow around the outer circumferential surface 226 of the injection needle 22, and therefore it is possible to allow the outer circumferential surface 226 of the injection needle 22 to be in close contact with the inner circumferential surface 232 of the insertion hole 231 formed in the needle fixing part 23. Therefore, the formulation does not accumulate between the injection needle 22 and the needle fixing part 23, and the formulation does not remain between the injection needle 22 and the needle fixing part 23. Accordingly, in the aforementioned case, the formulation contained in the barrel body can be used without waste. Further, when the formulation is expelled from the barrel body 21, there is no formulation flowing out not only around the recess but also around the gap between the injection needle 22 and the needle fixing part 23, and therefore the formulation can be rectified during the injection.

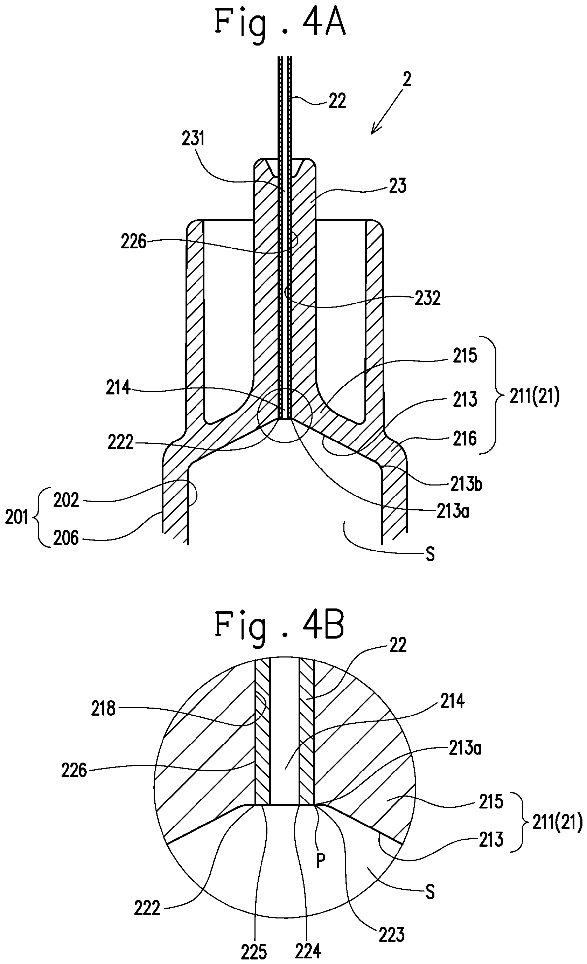

In the aforementioned embodiment, the case where the inner wall surface 213 of the end wall 211 expands from the center of the proximal end surface 225 of the injection needle 22 has been described, but there is no limitation to this. As shown in Fig. 4A and Fig. 4B, the inner wall surface 213 of the end wall 211 may expand from the outer circumferential edge 223 of the injection needle 22.

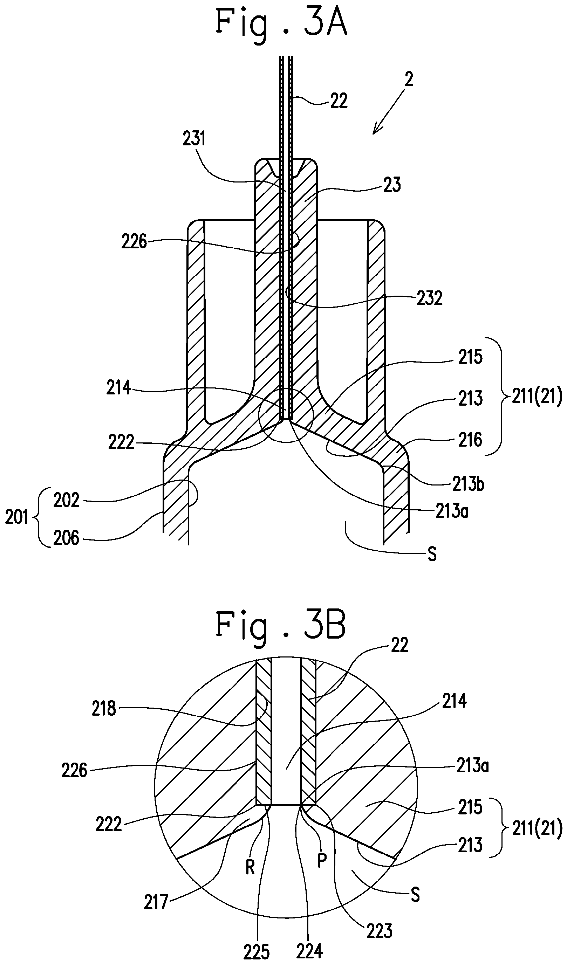

Further, as shown in Fig. 3A and Fig. 3B, the inner wall surface 213 of the end wall 211 may expand from the inner circumferential edge 224 of the injection needle 22 toward an inner wall surface 202 of the body wall 201. In this case, the entire region of the proximal end surface 225 of the injection needle 22 is covered by the inner wall surface 213 of the end wall 211. Accordingly, the barrel with needle 2 can allow the injection needle 22 to be inserted and attached more firmly.

In the aforementioned embodiment, the inner wall surface 213 of the end wall 211 is formed to be substantially flat from the inner circumferential edge 213a to the outer circumferential edge 213b, but there is no limitation to this. The inner wall surface 213 may be curved in the radial direction of the body wall 201 to be concave or convex. That is, the inner wall surface 213 of the end wall 211 may be an arcuate surface extending from the inner circumferential edge 213a to the outer circumferential edge 213b.

In the aforementioned embodiment, the case where the inner wall surface 213 of the end wall 211 expands in a direction intersecting the axial direction of the body wall 201 and inclined toward the other opening 205 side of the body wall 201 from a specific position between the outer circumferential edge 223 and the inner circumferential edge 224 has been described, but there is no limitation to this. The inner wall surface 213 of the end wall 211 may expand in the radial direction of the body wall 201. Further, as shown in Fig. 4A and Fig. 4B, the inner wall surface 213 of the end wall 211 may partially expand in the radial direction of the body wall 201 and expand continuously therewith in a direction intersecting the axial direction of the body wall 201 and inclined toward the other opening 205 side of the body wall 201.

In the aforementioned embodiment, the case where the end wall 211 is formed into a tapered shape has been described, but there is no limitation to this. For example, the end wall 211 may be formed stepwise. Further, it may have an uneven surface.

Further, the end edge of the end wall 211 may be elliptical, triangular, quadrangular, or the like. Following this, the body wall 201 may be formed into elliptical, triangular, and quadrangular cylindrical shapes and the like, corresponding to the shape of the end edge part 216 of the end wall 211.

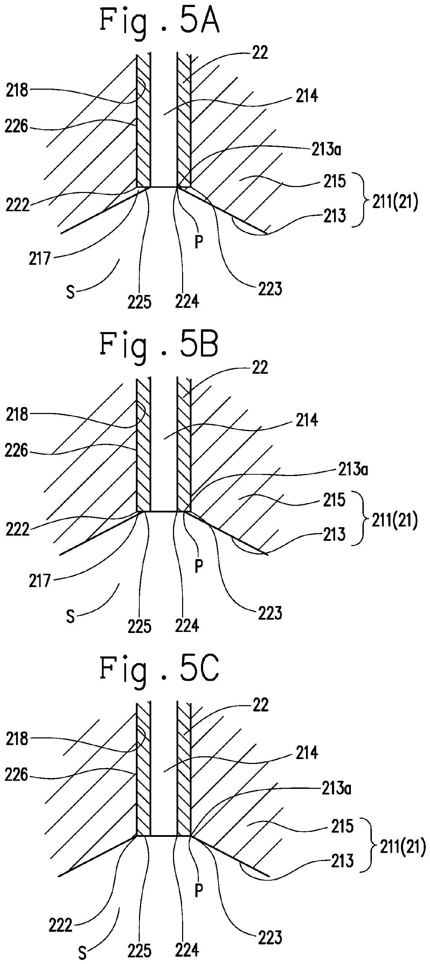

In the aforementioned embodiment, the case where the inner wall surface 213 of the end wall 211 constitutes the curved surface R from the connection point P with the injection needle 22 to the specific position has been described, but there is no limitation to this. As shown in Fig. 5A to Fig. 5C, the inner wall surface 213 of the end wall 211 may be formed straight from the connection point P with the injection needle 22 to the inner wall surface 202 of the body wall 201.

In the aforementioned embodiment, the case where the proximal end surface 225 of the injection needle 22 is constituted by a flat surface without unevenness has been described, but there is no limitation to this. The proximal end surface 225 of the injection needle 22 may be a convex surface projecting toward the other end 204 side of the body wall 201 or may be a concave surface recessed toward the distal end 221 side. Further, the proximal end surface 225 may be an uneven surface.

In the aforementioned embodiment, the case where the injection needle 22 is formed with a constant inner diameter and a constant outer diameter from the distal end 221 to the proximal end 222 has been described, but there is no limitation to this. The injection needle 22 may be tapered to have a larger diameter in the proximal end part than in the distal end part. Further, the injection needle 22 is not limited to one having a cylindrical shape, and may have a polygonal sectional shape such as a triangular shape.

In the aforementioned embodiment, there is no particular mention of the shape of the outer circumferential surface 226 of the injection needle 22, but the outer circumferential surface 226 of the injection needle 22 may be provided with a rib or a groove or may be provided with an uneven part formed by blasting. The uneven part may be formed from the proximal end surface 225 or may be formed from a position away from the proximal end surface 225 on the distal end 221 side.

In the aforementioned embodiment, the case where the end wall 211 is formed into a tapered shape, and therefore the distal end of the plunger 3 is formed into the tapered shape has been described, but there is no limitation to this. The distal end of the plunger 3 needs only to be formed in conformity with the shape of the inner wall surface 213 of the end wall 211. In the case where the end wall 211 is formed into a plate shape, and the inner wall surface 213 is not inclined toward the other opening 205 side of the body wall 201, it may be formed flat, accordingly.

### REFERENCE SIGNS LIST

1: Syringe
2: Barrel with needle
21: Barrel body
201: Body wall
202: Inner wall surface
203: One end
204: The other end
211: End wall
213: Inner wall surface
213a: Inner circumferential edge
213b: Outer circumferential edge
214: Insertion hole
215: Body
216: End edge part
217: Supporting part
218: Inner circumferential surface
22: Injection needle
221: Distal end
222: Proximal end
223: Outer circumferential edge
224: Inner circumferential edge
225: Proximal end surface
226: Outer circumferential surface
23: Needle fixing part
231: Insertion hole
232: Inner circumferential surface
3: Plunger
31: Expelling part
32: Plunger body
P: Connection point
S: Internal space
R: Curved surface

## Claims

1. A barrel with needle comprising:
a barrel body defining an internal space configured to contain a formulation; and
an injection needle with its proximal end opening into the internal space of the barrel body, wherein
the barrel body comprises: a cylindrical body wall; and an end wall closing one opening of the body wall and having an inner wall surface located on the body wall side,
the injection needle is inserted and attached to the end wall, and
the inner wall surface of the end wall expands in a radial direction of the body wall or in a direction intersecting an axial direction of the body wall and inclined toward the other opening side of the body wall, with any one of an outer circumferential edge, an inner circumferential edge, and a specific position between the outer circumferential edge and the inner circumferential edge on a proximal end surface of the injection needle serving as a starting point.

2. The barrel with needle according to claim 1, wherein
the inner wall surface of the end wall expands in a direction intersecting the axial direction of the body wall and inclined toward the other opening side of the body wall, from the inner circumferential edge or a specific position between the outer circumferential edge and the inner circumferential edge on the proximal end surface of the injection needle.

3. A syringe comprising:
the barrel with needle according to claim 1 or 2; and
a plunger configured to be inserted into the barrel with needle and to expel the formulation contained in the barrel body to the outside of the barrel with needle through a distal end of the injection needle.
